# EUROPEAN PATENT APPLICATION

(11) **EP 1 992 277 A1**
(43) Date of publication of application: **19.11.2008**
(21) Application number: 07301039.9
(22) Date of filing: 14.05.2007
(51) Int. Cl.: A61B 3/12

(54) **Optical device and method for acquiring images of eye structures**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Texier, Christian

(57) **Abstract**

The invention concerns an optical device for acquiring images of the eye structures, characterized in that it comprises:
- a light source (2);
- an endoscope connected to the light source (2), through an optical fibre, at one end for conducting and projecting an incident light beam (Bi) through a tip (10), said tip (10) being adapted to be applied nearby the cornea (7) of an eye (20) according to a given orientation;
- an imaging device connected to said endoscope (1) with said imaging device being adapted to acquire images of the eye structures from a light beam (Br) coming from the structures of the eye (20).

## Description

The present invention relates to medical imaging in the field of ophthalmology. In particular, it relates to means for acquiring images of eye structures of an animal or a human.

Imaging eye structures has become an essential step for exploring eye structures in the diagnostic and follow up of retinal diseases or lesions in humans and animals.

Imaging the fundus can for instance provide diagnostic of retinal detachment, retinal atrophy, and blood vascular occlusion whereas examination of the irido-corneal angle can explain increase intraocular pressure leading to glaucoma and ganglion cell degeneration.

However, contrast of these images is often affected by corneal reflections and the peripheral retina cannot be documented.

In order to overcome such a limitation, high resolution fundus imaging with viewing of the extreme periphery can be obtained with the scanning laser ophthalmoscope, but, such a technique is expensive and only allows monochromatic imaging.

In particular, eye examinations of the cornea, anterior chamber, lens and posterior chamber and vitreous are generally first achieved with a slit lamp apparatus. Images of the fundus can then be obtained using indirect ophtahlmoscopy or confocal scanning laser ophthalmoscopes.

It is also possible to counteract the corneal refraction by optical applanation allowing the observation of the fundus of any eye, but with a narrow field of view.

The present invention provides a method and a corresponding device to increase the quality of images of the eye structures and, in particular, to improve the contrast of such images.

Thus, the invention concerns an optical device for acquiring images of the eye structures, characterized in that it comprises: a light source; an endoscope connected to the light source, through an optical fibre, at one end for conducting and projecting an incident light beam through the tip, said tip being adapted to be applied nearby the cornea of an eye according to a given orientation; an imaging device connected to said endoscope through said imaging device being adapted to acquired imaged of the eye structures from a light beam coming from the structures of the eye.

The device of the invention may present one or more of the following features:
- the tip of the endoscope is suitable to project a circular-shaped illumination;
- the tip of the endoscope is suitable to project a lateral crescent-shaped illumination;
- the field of view of the endoscope and of the imaging device are congruent;
- the light source is suitable to provide an incident beam with an adjustable and/or limited intensity;
- the light source is a cold light source or an infrared source;
- the endoscope is a rigid endoscope preferably an otoscope;
- at least one filter is applied between the endoscope and the light source;
- the imaging device is a video camera or a digital camera;
- the endoscope is coupled with a Laser;
- a film is disposed on the tip of the endoscope;
- a designed single lens having designed positions for orienting the tip of the endoscope;
- the filters are barrier filters or UV filters or fluorescence filters.

Furthermore, the invention concerns a method for acquiring images of the eye structures, characterized in that it comprises the following steps: an application, according to a given orientation, of the tip of an endoscope nearby the cornea of an eye, said endoscope being connected to a light source through an optical fibre at one end for conducting and projecting an incident light beam through the tip of the endoscope; an acquisition of images of the eye structures from light coming from the structures of the eye by means of an imaging device connected to said endoscope.

The method of the invention may present one or more of the following features:
- during the acquisition reflected beams on the fundus are acquired;
- during the acquisition, fluorescence of the eye tissue is acquired.
- the step of the application of the tip of the endoscope is implemented by means of an endoscope having a circular-shaped illumination;
- the step of the application of the tip of the endoscope is implemented by means of an endoscope having a lateral crescent-shaped illumination;
- previously to the application of the tip of the endoscope, a gel is applied on the cornea of the eye, and in that the tip of the endoscope is applied into the gel;
- the tip of the endoscope is in contact with the cornea of the eye;
- during the step of acquisition, at least one burst of a plurality of images is acquired.
- the acquired images are transferred from the imaging device to a computer in order to be processed.
- the images are processed by adjusting contrast and/or luminosity.
- the images are acquired on an eye of a mouse, a cat, a dog, a monkey, a lamb, or a human.

The invention provides a low cost and a simply device to image the eye structures.

Other features and advantages of the invention will appear in the following description. Embodiments of the invention will be described with references to the drawings, in which:
- Figure 1 illustrates the means used to implement the method of the invention;
- Figure 2 illustrates two configurations with different orientations of the tip of an endoscope used in the method of the invention;
- Figure 3a and Figure 3b respectively illustrate a perspective view of the tip of the endoscope and a side view of the tip of the endoscope according to the axis CC';
- Figure 4a, 4b and 4c illustrate fundus images of a mouse obtained with the method of the invention;
- Figure 5a, 5b,5c show an illustration of optical aberrations in the method of the invention;
- Figures 6a, 6b, 6c and 6d show the reflectance of specific fundus structures;
- Figures 7a, 7b, 7c, 7d, 7e, 7f, 7g and 7h show the variation in contrast of human retinal vessels with increased intensity of incident light;
- Figure 8a and 8b show images of the peripheral retina and the ciliary body of an albino mouse;
- Figure 9 shows an image of the irido-corneal angle of the eye of the cat;
- Figure 10 shows an angiogram obtained with the invention.

Surprisingly, Applicant has observed that an application of an illuminating endoscope nearby a cornea of an eye allows wide field fundus imaging.

Such application is possible because the diameter of the tip of the endoscope is equal or inferior to the diameter of the pupil.

A fundus image can be obtained by placing an endoscope nearby the cornea, as long as the outer diameter does not exceed the diameter of the dilated pupil, that is, 3 to 4 mm in a mouse.

Classically, an endoscope is used to observe the interior surfaces of an organ by inserting a tube into the body.

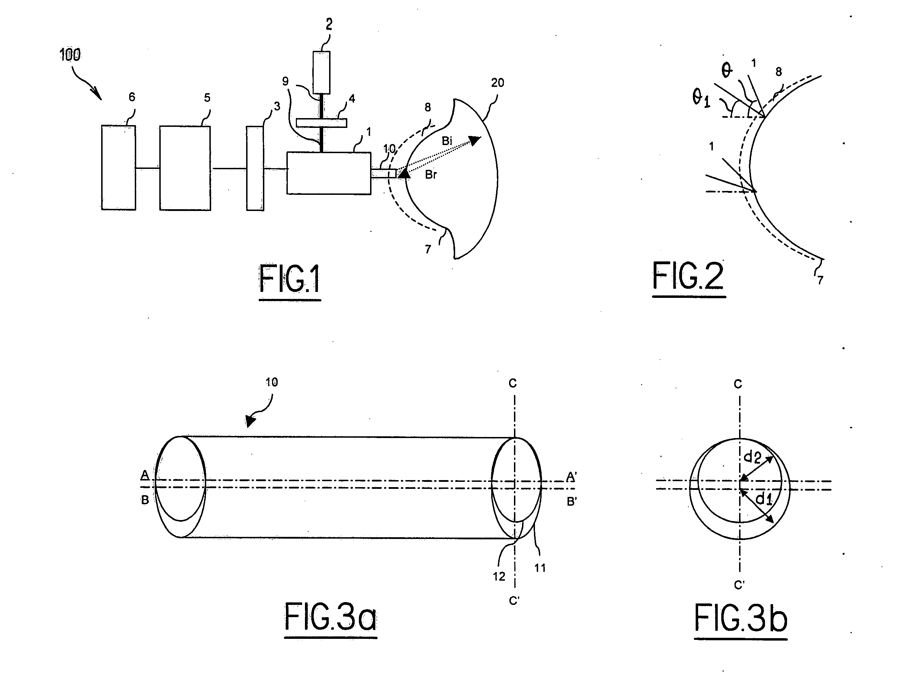

Figure 1 shows an optical device used to implement a method for acquiring images of the structures of the eye.

The optical device comprises an endoscope 1 with parallel illumination and observation channels, connected to an imaging device 5 such as a photographic camera (to acquire images) or a video camera (for screening procedures), through an adapter 3 containing a +5 lens to acquire images.

Notice that, to acquire images, the field of view of the endoscope 1 and of the imaging device 5 are congruent.

As imaging device 5, a reflex digital camera with a 6.1 million pixels Charged Couple Device (CCD) image sensor is preferably used for increasing the definition.

As a rule, each pixel of a CCD codes corresponds to three color channels (RGB mode), that is, Red (peak approximately equal to 610 nm), Green (peak approximately equal to 550 nm) and Blue (peak approximately equal to 470nm), with a certain degree of overlapping between channels. (the resolution is better when necessary in black and white because 4 pixels in grey).

The endoscope 1 is connected to a light source 2, preferably a cold light source 2 for instance, a xenon lamp, through a flexible optical fibre 9.

The light source 2 can be an infrared source.

The light source 2 is further suitable to provide an incident light beam with an adjustable intensity. Such adjustability permits the protection of the retina of the eye.

The endoscope 1 is a rigid endoscope and advantageously an otoscope.

The method of the invention performed with the optical device of the invention comprises an application of the tip 10 of the endoscope 1 nearby the cornea 7 of the eye 20.

According to a preferred embodiment, a gel 8 is applied on the cornea 7 of the eye 20; the gel 8 is for instance methylcellulose. The tip 10 of the endoscope 1 is then applied into the gel 8.

Additionally for sterility problems, a single use lens can be applied on the cornea or a transparent film can be inserted on the tip of the endoscope.

The contact with the cornea 7 is not essential, but advantageously decreases parasitic reflections.

It can be noticed that the tip 10 of the endoscope 1 is applied according to a given orientation θ₁, θ₂, etc.

Advantageously, the possibility to orient the tip 10 of the endoscope 1 under large angles with respect to the eye central optic permits to focus on the eye periphery.

A lens can also be used to prepare interesting orientations by having designed positions for the tip of the endoscope.

Figure 2 illustrates two configurations with different orientations of the tip 10 of the endoscope 1 applied into the gel 8.

Although the use of classical endoscope permits to image the eye structures, Applicant has observed that the use of a classical endoscope, with a circle-shaped illumination at this tip is not sufficient to ensure accurate viewing of the fundus. In particular, the contrast of the images is not sufficient to accurately observe the fundus.

A classical endoscope comprises a first cylinder co-axial with a second cylinder having a diameter inferior to the diameter of the first cylinder. These two cylinders provide a circle-shaped illumination at the tip of such endoscope, and the light is emitted through the tip projecting a circle-shaped incident light beam on the eye.

As known, the second cylinder comprises means to acquire light beam from the area of interest.

With this disposition, Gullstrand's principles of separation of the illumination and observation axis are achieved ensuring viewing of the fundus.

Figures 3a and 3b show different views of the endoscope preferably used in the invention.

Figure 3a shows a perspective view of the tip 10 of the endoscope 1 and Figure 3b shows a side view of the tip 10 according to the axis CC' of the endoscope 1.

The tip 10 of the endoscope 1 comprises a first cylinder 11 having a first diameter d1, and a second cylinder 12, which is non coaxial with the first cylinder 11, having a second diameter d₂ inferior to the first diameter d₁.

These two cylinders 11, 12 provide a crescent-shaped tip 10, and the light is emitted through the tip 10 projecting a lateral crescent-shaped incident light beam Bi.

This illumination involves a minimization of axial reflections.

The endoscope 1 has for instance, a 5cm length, 3mm outer diameter d1 with step index lenses that have an angle of view of 0°, a field of view of 80° in the air, and a crescent illuminating tip 10.

The acquired images are obtained by measuring the light beam Br coming from the eye. They may be transferred to a computer 6 which processes them, in particular, for adjusting their contrast and/or their luminosity.

In particular, in order to obtain images of the fundus 20, the reflected light on the fundus is acquired.

Also, in order to obtain a fluorescent angiogram, the fluorescent emitted by the dye is acquired.

Additionally, filters 4 can be placed between the light source 2 and the endoscope 1.

These filters are used to control the light source 2 for UV exclusion and thus protecting eye tissues 20.

During the acquisition, at least one burst of a plurality of images is acquired to select the best acquired images. Advantageously, each burst comprises four images.

With the method of the invention and the corresponding optical device, acquisitions of fundus images are possible with high quality and good contrast in comparison with usual techniques.

The acquired images can be used for a diagnostic method which may be implemented in an additional step after the method performed with the optical device for acquiring images of the eye structures in accordance with the present invention.

The definition of the acquired images depends on the imaging device. The acquired images shown in this description have an estimated definition of the image acquisition system (e.g., 1.9 million pixels).

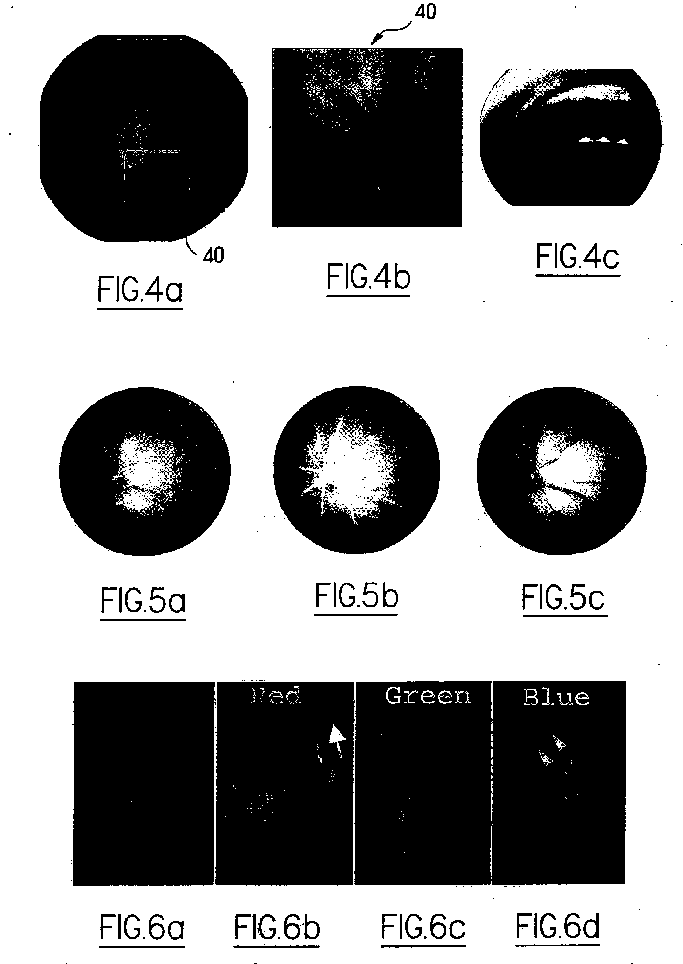

Examples of fundus images of a mouse are shown in figure 4a, 4b and 4c.

On these figures the shadow of retinal vessels on the Retinal Pigment Epithelium (RPE), which is due to the lateral disposition of the illumination channel provided with the invention, is remarkable.

Figure 4a shows a view of a posterior pole. The scale is given by the optical nerve 40, which is approximately 220 microns in diameter.

Figure 4b shows a magnification of figure 4a. In other words it is a magnification of the optical nerve 40 and vessels of Figure 4a.

This figure accurately shows the delineation of the contour of the optical nerve 40 and the nerve fibre layer.

Figure 4c shows a view of the peripheral retina and ciliary body which is acquired by changing the orientation of the endoscope, in particular by placing the endoscope perpendicularly to the limbus.

This disposition allows imaging of the peripheral retina and the ciliary body.

Furthermore, the presence of the peripheral longitudinal vein (see arrowheads) and the absence of significant spherical aberrations as compared to the view of the posterior pole (see Figure 4a) are of great interest.

Figure 5a, 5b, 5c show an illustration of optical aberrations encountered in the images acquired with the method of the invention.

Figure 5a shows a color fundus image, figure 5b shows a monochromatic fundus image acquired through the red channel, and figure 5c shows a monochromatic fundus image acquired through the blue channel.

In the blue case (Figure 5c), the field of view is large, peripheral vessels 50 are visible.

Furthermore, a chromatic and/or spherical aberration is suggested by the larger field of view for shorter wavelengths. Blue light images have indeed a larger field than red light images. This is consistent with the stronger dispersion of a blue incident light.

Figures 6a, 6b, 6c and 6d show the reflectance of specific fundus structures.

Figure 6a shows a color fundus image, Figures 6b, 6c and 6d show monochromatic fundus images acquired through the red, green, and blue channel respectively.

In the red light channel image, the axial reflex over arterioles and venules and the visualization of a pigment defect 60 can be seen.

Red monochromatic imaging enhances the contrast of pigment alterations, as well as the axial reflex from vessels.

Green monochromatic imaging enhances the contrast of veins, while arteries remain relatively isoreflective to the surrounding background.

The blue channel image provides the best contrast for both arteries and veins; in particular, the best contrast for retinal vessels over the choroids 61 is obtained.

The reflectance of the nerve fibre layer is more evidenced in blue or green monochromatic images.

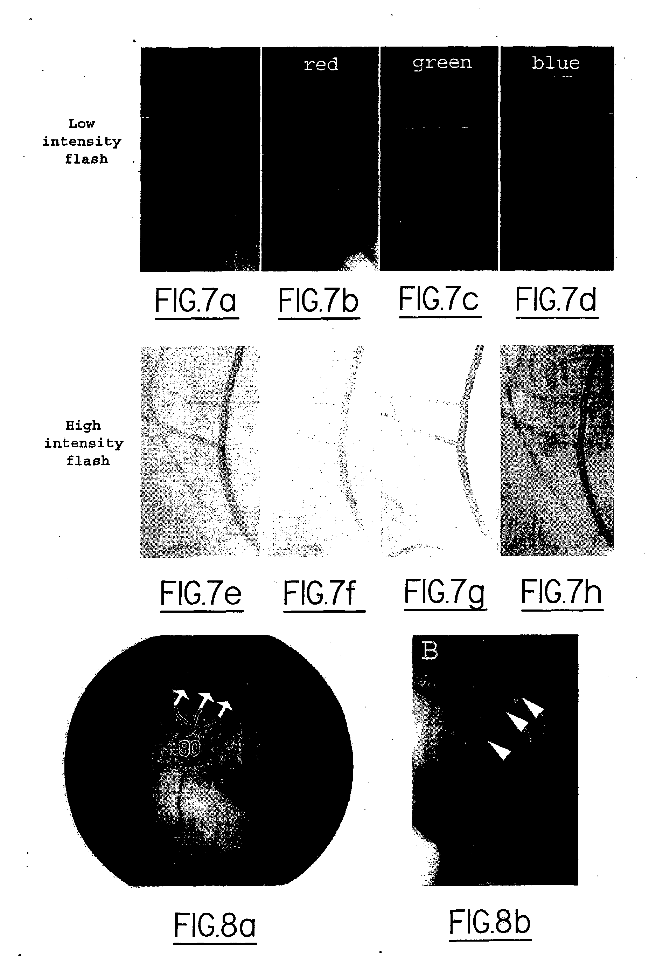

Figures 7a, 7b, 7c, 7d, 7e, 7f, 7g and 7h show the variation in contrast of human retinal vessels with increased intensity of incident light.

Figures 7a and 7e show color images, Figures 7b and 7f show red monochromatic images, Figures 7c and 7g show green monochromatic images, Figures 7d and 7h show blue monochromatic images.

It can be noticed that, for animal and human eyes, filtering UV light and limiting light intensities of the incident light is required during examination and image acquisition.

In particular, in order to compensate differences in fundus pigmentation in illumination intensity and in vessel size, fundus images of a pigmented human subject acquired with a conventional fundus camera (Topcon 50 IA from Topcon Corporation, Tokyo, Japan) at different flash intensities is magnified in order to visualize small retinal vessels (i.e. in the magnitude of 40-60 microns).

In particular, fundus images in a pigmented subject are acquired with a normal flash intensity (Figures 7a, 7b, 7c and 7d) and with high intensity flash (Figures 7e, 7f, 7g and 7h).

This shows that, while in low flash intensity the best contrast for arterioles is obtained with green light, increasing the flash intensity increases the axial reflection from arterioles as well as the amount of light backscattered from the choroids/RPE, thus altering the contrast of arterioles. This loss of contrast is much less marked with blue light.

It can be noticed that the axial light reflection of veins is faintly visible with a low intensity flash but clearly visible with a high intensity flash.

Figure 8a shows color image of the peripheral retina and the ciliary body 90 of an albino mouse.

The overall organization of the ciliary body venous drainage into the vortex veins is discernable.

Figure 8b shows a monochromatic high magnification image of Figure 8a. In this figure, the venous drainage of individual ciliary body 91 is clearly visible.

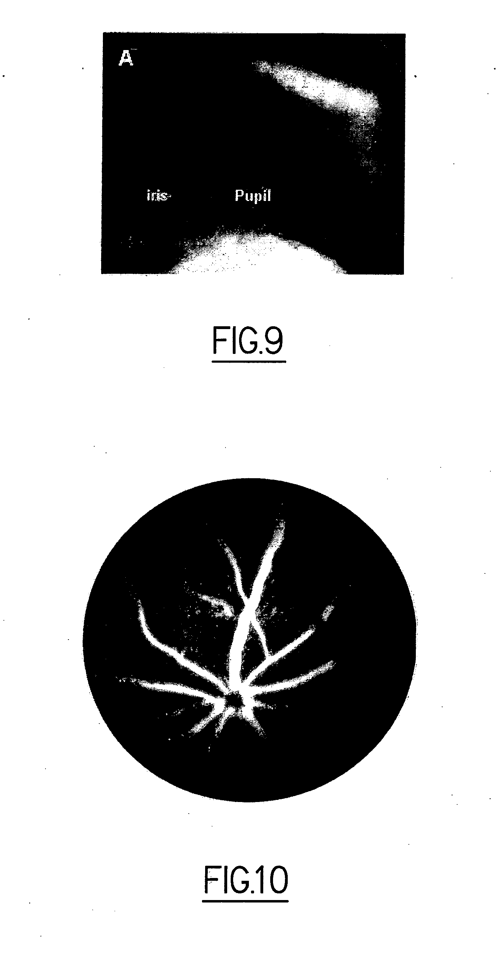

Figure 9 show images of the irido-corneal angle in various species.

When the tip 10 of the endoscope 1 is oriented on the cornea a wide angle with the eye optic axis is obtained, the irido-corneal angle can be then imaged with great precision.

Visualization of the irido-corneal angle is generally performed with the help of goniolens which enables to have a circular view of irido-corneal angle.

The irido-corneal angle is advantageously observed with enlarged view of the pectinate ligament at the level of the trabeculum.

These observations facilitate the diagnostic of glaucoma especially acute glaucoma with a closed angle. It could also be used to examine the presence of anterior irido-corneal synechia, a risk factor for chronic glaucoma.

Coupling an argon laser to the endoscope provides an easier way to achieve trabeculum retraction than with the current argon laser coupled to the slit lamp and using the goniolens.

Therefore, the method and its corresponding device offers a new approach to visualize the irido-corneal angle with great details and eventually to treat the trabeculum more easily thanks to the easiness to orient the tip of the endoscope.

More generally, other lasers could also be coupled to the endoscope 1 like a YAG laser.

Figure 10 shows such a fluorescein angiogram obtained on a pigmented mouse after interposition of adequate excitation and barrier filters. The method can therefore be used to obtain fluorescent images of the different eye structures.

The invention allows high resolution wide field digital fundus imaging in the mouse eye.

Of course the invention can be used with human eye or any other animals, such as cats, dogs, monkeys, lambs, etc.

The invention significantly improves the quality of in vivo images of retina and ciliary body.

Although resolution is affected by chromatic aberration, resolution can be improved by monochromatic imaging.

Furthermore, compared with current eye structures imaging techniques, specific advantages of the invention are the high contrast due to the absence of corneal reflections and to the lateral illumination, and the possibility to image the extreme periphery.

Known materials providing good quality images of the structures of the eye are heavy equipments that are relatively expensive and difficult to move. This is particularly true for the scanning laser ophthalmoscope which requires laser alignment after each move. Furthermore, patients have to sit in front of these equipments which is not always easy for infants, highly handicapped or elderly persons.

By contrast, all the required pieces for the optical device of the invention fit in a suitcase and can work on a battery. Furthermore, the endoscope probe can be handled in any position depending on the patient situation for the eye examination.

Also the optical device of the invention is suitable to obtain images of the fundus and images of the irido-corneal angle with only one device which is a very advantageous property.

Therefore, this is a low-cost and portable device which is very adapted not only for research laboratories and ophthalmology clinic but also to perform complete ophthalmologic exams in remote areas.

## Claims

1. Optical device for acquiring images of the eye structures, **characterized in that** it comprises:
- a light source (2);
- an endoscope connected to the light source (2), through an optical fibre, at one end for conducting and projecting an incident light beam (Bi) through the tip (10), said tip (10) being adapted to be applied nearby the cornea (7) of an eye (20) according to a given orientation;
- an imaging device connected to said endoscope (1) through said imaging device being adapted to acquired imaged of the eye structures from a light beam (Br) coming from the structures of the eye (20).

2. Optical device according to claim 1, **characterized in that** the tip (10) of the endoscope (1) is suitable to project a circular-shaped illumination.

3. Optical device according to claim 1, **characterized in that** the tip (10) of the endoscope (1) is suitable to project a lateral crescent-shaped illumination.

4. Optical device according to any one of claims 1 to 3, **characterized in that** the field of view of the endoscope (1) and of the imaging device (5) are congruent.

5. Optical device according to any one of claims 1 to 4, **characterized in that** the light source (2) is suitable to provide an incident beam (Bi) with an adjustable and/or limited intensity.

6. Optical device according to any one of claims 1 to 5, **characterized in that** the light source (2) is a cold light source or an infrared source.

7. Optical device according to any one of claims 1 to 6, **characterized in that** the endoscope (1) is a rigid endoscope preferably an otoscope.

8. Optical device according to any one of claims 1 to 7, **characterized in that** at least one filter (4) is applied between the endoscope (1) and the light source (2).

9. Optical device according to any one of claims 1 to 8, **characterized in that** the imaging device (5) is a video camera or a digital camera.

10. Optical device according to any one of claims 1 to 9, **characterized in that** the endoscope (1) is coupled with a Laser.

11. Optical device according to any one of claims 1 to 10, **characterized in that** a film is disposed on the tip (10) of the endoscope (1).

12. Optical device according to any one of claims 1 to 11, **characterized in that** a designed single lens having designed positions for orienting the tip (10) of the endoscope (1).

13. Optical device according to claim 8, **characterized in that** the filters are barrier filters or UV filters or fluorescence filters.

14. Method for acquiring images of the eye structures, **characterized in that** it comprises the following steps:
- an application, according to a given orientation (θ1), of the tip (10) of an endoscope (1) nearby the cornea (7) of an eye (20), said endoscope (1) being connected to a light source through an optical fibre (9) at one end for conducting and projecting an incident light beam (Bi) through the tip (10) of the endoscope (1);
- an acquisition of images of the eye structures from light (Br) coming from the structures of the eye (20) by means of an imaging device (5) connected to said endoscope (1).

15. Method according to claim 14, **characterized in that** during the acquisition reflected beams on the fundus (20) are acquired.

16. Method according to any one of claims 13 to 14, **characterized in that** during the acquisition, fluorescence of the eye tissue is acquired.

17. Method according to any one of claims 14 to 16, **characterized in that** the step of the application of the tip (10) of the endoscope (1) is implemented by means of an endoscope (1) having a circular-shaped illumination.

18. Method according to claim 14, **characterized in that** the step of the application of the tip (10) of the endoscope (1) is implemented by means of an endoscope (1) having a lateral crescent-shaped illumination.

19. Method according to any one of claims 14 to 18, **characterized in that**, previously to the application of the tip (10) of the endoscope (1), a gel (8) is applied on the cornea (7) of the eye, and **in that** the tip (10) of the endoscope (1) is applied into the gel (8).

20. Method according to any one of claims 14 to 19, **characterized in that** the tip (10) of the endoscope (1) is in contact with the cornea of the eye.

21. Method according to claim 14, **characterized in that** during the step of acquisition, at least one burst of a plurality of images is acquired.

22. Method according to claim 21, **characterized in that** the acquired images are transferred from the imaging device (5) to a computer (6) in order to be processed.

23. Method according to claim 22, **characterized in that** the images are processed by adjusting contrast and/or luminosity.

24. Method according to any one of claims 14 to 23, **characterized in that** the images are acquired on an eye of a mouse, a cat, a dog, a monkey, a lamb, or a human.
